# EUROPEAN PATENT APPLICATION

(11) **EP 4 252 830 A1**
(43) Date of publication of application: **04.10.2023**
(21) Application number: 21909303.6
(22) Date of filing: 17.12.2021
(51) Int. Cl.: A61N 1/362

(54) **WIRE-FREE PACEMAKER, AND HEAD-END COMPONENT AND TAIL-END COMPONENT THEREOF**

(30) Priority: 25.12.2020 CN 202011564889
(71) Applicant: MicroPort Soaring CRM (Shanghai) Co., Ltd., Free Trade Zone (Shanghai) (CN)
(72) Inventor: HE, Mingchen, Shanghai 201203 (CN)
(74) Representative: Marks & Clerk LLP
(86) International application number: PCT/CN2021/139373
(87) International publication number: WO 2022/135307

(57) **Abstract**

A leadless pacemaker, a leading component and a trailing component are disclosed. The leading component includes a leading body and a locking member, which are coupled to each other. The leading body is configured to be secured at a predetermined site. The locking member assumes an initial configuration when there is no external force acting thereon. At least part of the locking member is expandable or contractible radially with respect to the leading body under the action of an external force. The locking member defines an accommodating chamber extending in an axial direction of the leading body. The accommodating chamber includes a main portion and a diametrically-reduced portion located at a proximal end of the main portion in the axial direction of the leading body. In the initial configuration of the locking member, an inner radial dimension of the diametrically-reduced portion is smaller than an inner radial dimension of the main portion. The diametrically-reduced portion is configured to accommodate a waist section of the trailing component, and the main portion is configured to accommodate a first shoulder section of the trailing component. The trailing component can be conveniently inserted into and removed from the accommodating chamber, enabling convenient replacement of the trailing component.

## Description

### TECHNICAL FIELD

The present invention relates to the field of medical devices and, in particular, to a leadless pacemaker, a leading component and a trailing component.

### BACKGROUND

Cardiac pacemaker is an implantable therapeutic electronic device. It incorporates an impulse generator for delivering electrical impulses which are transferred via leads to electrodes to stimulate the heart muscle that the electrodes are attached to, thus causing the heart to beat and contract and effecting treatment of heart dysfunction caused by some cardiac arrhythmia conditions. However, traditional lead cardiac pacemakers require the creation of pockets where they are to be inserted and long-term presence of their leads in veins and therefore suffer from a high incidence of pocket- or lead-related complications, which present a serious threat to the life and health of patients. By contrast, leadless pacemakers do not need electrode wires and integrate a battery, circuitry and pacing electrodes into a "compact capsule" that can be overall implanted into the heart simply via a percutaneous catheter, and have a wide range of advantages including ease of operation, high convenience, minimal trauma, eliminated need for surgical creation of a pacemaker pocket, unaffected patients' appearance and absence of pacemaker pocket- or lead-related complications. Therefore, the leadless pacemakers have found extensive use in clinical practice.

A conventional leadless pacemaker typically includes a fixation feature disposed at its leading end, which can be screwed into the myocardium when the pacemaker has been delivered through a delivery sheath to a target site within the heart, thus attaching the pacemaker to the myocardium. However, on the other hand, operations involved in the implantation of such a leadless pacemaker are challenging and associated with the problems of easy dislodgement and possible damage to the myocardium. In particular, dislodgement within two months after implantation has been frequently reported. On the other hand, after implantation, the fixation feature of the leadless pacemaker will be wrapped or encapsulated by myocardial tissue, making the leadless pacemaker difficult to retrieve. Therefore, when its battery runs out, a new pacemaker has to be implanted, with the old one having to be still retained in the heart. Consequently, in addition to continued occupation of the intracardiac space, the old leadless pacemaker with the depleted battery may adversely affect the new pacemaker. Due to the limited intraventricular space, at most two leadless pacemakers can be deployed therein. As a result, the conventional leadless pacemakers are generally only used in older patients and lack general applicability. Further, the implantation of the new pacemaker may require the creation of another incision in the heart, bringing additional damage to the patient.

### SUMMARY OF THE INVENTION

It is an objective of the present invention to overcome the problem of inconvenient replacement of existing leadless pacemakers after the expiry of their service life, by presenting a novel leadless pacemaker, leading component and trailing component.

To this end, in a first aspect of the present invention, there is provided a leading component of a leadless pacemaker, which is adapted to be detachably coupled to a trailing component of a leadless pacemaker. The leading component includes a leading body and a locking member, which are coupled to each other.

The leading body is configured to be secured at a predetermined site.

The locking member assumes an initial configuration when there is no external force acting thereon, and at least part of the locking member is expandable or contractible radially with respect to the leading body under the action of an external force.

The locking member defines an accommodating chamber extending in an axial direction of the leading body, which includes a main portion and a diametrically-reduced portion located at a proximal end of the main portion in the axial direction of the leading body. The diametrically-reduced portion has an inner radial dimension smaller than an inner radial dimension of the main portion when the locking member is in the initial configuration. The diametrically-reduced portion is configured to accommodate a waist section of the trailing component, and the main portion is configured to accommodate a first shoulder section of the trailing component.

Optionally, the locking member may include a first resilient element, a plurality of resilient strips and a plurality of resilient element connectors, the plurality of resilient strips arranged circumferentially around the leading body, the resilient strips fixedly coupled at their distal ends to the leading body, the resilient element connectors provided on inner sides of the resilient strips in such a manner that each adjacent two of the resilient element connectors are connected by the first resilient element, the plurality of the resilient element connectors defining the diametrically-reduced portion, portions of the plurality of resilient strips located at distal ends of the resilient element connectors defining the main portion.

Optionally, each of the resilient element connectors may include a first bevel and a second bevel, the first bevel facing proximally, the second bevel facing distally, both the first bevel and the second bevel inclined toward an axis of the leading body.

Optionally, each of the resilient element connectors may further include a first transition surface, the first transition surface transitionally joined to both the first bevel and the second bevel.

Optionally, each of the resilient element connectors may define a through bore extending therethrough in the circumferential direction of the leading body, the through bore configured for passage of the first resilient element therethrough, wherein the first resilient element is ring-shaped and passed successively through the through bores of the plurality of the resilient element connectors.

Optionally, the resilient element connectors may be provided on proximal ends of the resilient strips.

Optionally, three or four resilient strips may be provided, the resilient element connectors in one-to-one correspondence with the resilient strips.

Optionally, the leading body distally may define a pointed helix, wherein a proximal end of the leading body is provided with an electrode head, the electrode head electrically connected to the pointed helix, the electrode head configured for electrical connection with an electrode connector in the trailing component.

Optionally, the electrode head may be columnar and extend in the axial direction of the leading body.

Optionally, when the locking member is in the initial configuration, the inner radial dimension of the diametrically-reduced portion may be smaller than an outer radial dimension of the waist section in the trailing component.

In a second aspect of the present invention, there is provided a trailing component of a leadless pacemaker, which is adapted to be detachably coupled to a leading component of a leadless pacemaker. The trailing component includes a waist section and a first shoulder section, which are coupled to each other.

The first shoulder section is located distally with respect to the waist section which has an outer radial dimension smaller than an outer radial dimension of the first shoulder section.

The first shoulder section is configured to be inserted through a diametrically-reduced portion of the leading component and accommodated in a main portion of the leading component and to be removed from the main portion through the diametrically-reduced portion of the leading component. The waist section is configured to be inserted and accommodated in the diametrically-reduced portion of the leading component and to be removed from the diametrically-reduced portion of the leading component.

Optionally, the first shoulder section may include a second transition surface joined to both a circumferential side wall of the first shoulder section and a distal end face of the first shoulder section.

Optionally, the trailing component may further include an electrode connector configured to be electrically connected to an electrode head of the leading component.

Optionally, the electrode connector may include an electrode chamber extending in an axial direction of the first shoulder section, the electrode chamber open distally and configured to accommodate the electrode head of the leading component.

Optionally, the electrode connector may further include a second resilient element arranged circumferentially around the electrode chamber, the second resilient element configured to abut against and thereby be electrically connected to the electrode head of the leading component.

Optionally, the second resilient element may be ring-shaped and have an inner radial dimension smaller than an outer radial dimension of the electrode head of the leading component.

Optionally, the trailing component may further include a second shoulder section coupled to a proximal end of the waist section, the second shoulder section having an outer radial dimension greater than the outer radial dimension of the waist section.

Optionally, the outer radial dimension of the second shoulder section may be equal to the outer radial dimension of the first shoulder section.

Optionally, the trailing component may further include a delivery connector disposed at a proximal end of the trailing component, the delivery connector configured for connection of a delivery device.

In a third aspect of the present invention, there is provided a leadless pacemaker, including the leading component as defined above and the trailing component as defined above. The trailing component is adapted to be detachably coupled to the leading component.

The outer radial dimension of the waist section is greater than the inner radial dimension of the diametrically-reduced portion when the locking member is in the initial configuration.

When the first shoulder section passes through the diametrically-reduced portion, the first shoulder section pushes and radially expands at least part of the locking member, and after the first shoulder section enters or is proximally removed from the main portion through the diametrically-reduced portion, the at least part of the locking member is radially contractible.

As a result of the first shoulder section entering the main portion, the waist section is inserted and accommodated in the diametrically-reduced portion.

In summary, the present invention provides a leadless pacemaker, a leading component and a trailing component. The leading component includes a leading body and a locking member, which are coupled to each other. The leading body is adapted to be secured to a predetermined object. The locking member assumes an initial configuration when there is no external force acting thereon. At least part of the locking member is expandable or contractible radially with respect to the leading body under the action of an external force. The locking member defines an accommodating chamber extending in an axial direction of the leading body. The accommodating chamber includes a main portion and a diametrically-reduced portion located at a proximal end of the main portion in the axial direction of the leading body. In the initial configuration of the locking member, an inner radial dimension of the diametrically-reduced portion is smaller than an inner radial dimension of the main portion. The diametrically-reduced portion is adapted to accommodate a waist section of the trailing component, and the main portion is adapted to accommodate a first shoulder section of the trailing component.

With this arrangement, the leading component and the trailing component are assembled in a detachable and separable manner. During use, the leading component may be first implanted within the heart at a predetermined site, and the trailing component may be delivered into the heart and coupled to and assembled with the leading component. When the service life of the leadless pacemaker expires, the trailing component may be disassembled from the leading component, the trailing component may be retrieved and the leading component may be retained at the predetermined site. After that, the trailing component may be replaced with a new one.

The locking member in the leading component enables the trailing component to be conveniently inserted into or removed from the accommodating chamber. In this way, the trailing component can be replaced almost in a non-invasive fashion, solving the problem of inconvenient replacement of existing leadless pacemakers after the expiry of their service life. Additionally, since the locking member is radially contractible, during delivery of the leading component, the locking member can be crimped by a sheath tube, allowing the leading component to have a reduced maximum outer diameter and hence increased passage ability. Additionally, the diametrically-reduced portion, the main portion, the waist section and the first shoulder section can be assembled together in such a manner that there are no protrusions on an exterior circumferential surface of the trailing component, which may cause damage to a blood vessel. As a result, the trailing component can be delivered without use of a protective sleeve, resulting in an additional increase in the passage ability of the trailing component.

### BRIEF DESCRIPTION OF THE DRAWINGS

Those of ordinary skill in the art would appreciate that the following drawings are presented merely to enable a better understanding of the present invention rather than to limit the scope thereof in any sense, in which:
Fig. 1 is a schematic diagram showing an implantation application of a leadless pacemaker according to an embodiment of the present invention;
Fig. 2 is a schematic assembly view of a leadless pacemaker according to an embodiment of the present invention;
Fig. 3 is a schematic illustration of a leading component according to an embodiment of the present invention;
Fig. 4 schematically illustrates resilient strips and resilient element connectors according to an embodiment of the present invention;
Fig. 5 is a schematic exploded view of a leading body according to an embodiment of the present invention;
Fig. 6 is a schematic illustration of a trailing component according to an embodiment of the present invention;
Fig. 7 is a schematic partial cross-sectional view of a trailing component according to an embodiment of the present invention;
Fig. 8 is a schematic axial cross-sectional view of a battery assembly according to an embodiment of the present invention;
Fig. 9 is a schematic exploded view of a battery assembly according to an embodiment of the present invention;
Fig. 10 is a schematic exploded view of an electronic assembly according to an embodiment of the present invention;
Fig. 11 is a schematic exploded view of an electrode connector according to an embodiment of the present invention; and
Fig. 12 is a schematic axial cross-sectional view of an electrode connector according to an embodiment of the present invention.

In these figures,
10-leading component; 11-leading body; 111-electrode head; 112-electrode; 113-stop ring; 114-stop notch; 115-snap ring; 116-pointed helix; 117-drug plunger; 12-locking member; 121-first resilient element; 122-resilient strip; 123-resilient element connector; 124-first bevel; 125-second bevel; 126-first transition surface; 127-through bore; 128-folding section; 13-accommodating chamber; 131-diametrically-reduced portion; 132-main portion;
20-trailing component; 201-first shoulder section; 202-waist section; 203-second shoulder section; 21-electrode connector; 211-third cover; 212-feedthrough; 213-intermediate element; 214-square body; 215-second resilient element; 216-second transition surface; 217-third bevel; 22-electronic assembly; 221-first casing; 222-circuit module; 23-first cover; 24-second cover; 25-battery assembly; 251-second casing; 252-battery; 253-power cable; 26-delivery connector;
30-delivery device;
50-heart.

### DETAILED DESCRIPTION

Objects, features and advantages of the present invention will become more apparent upon reading the following more detailed description of the present invention, which is set forth by way of particular embodiments with reference to the accompanying drawings. Note that the figures are provided in a very simplified form not necessarily drawn to exact scale for the only purpose of helping to explain the disclosed embodiments in a more convenient and clearer way. In addition, structures shown in the figures are usually part of actual structures. In particular, as the figures tend to have distinct emphases, they are often drawn to different scales.

As used herein, the singular forms "a", "an" and "the" include plural referents, unless the context clearly dictates otherwise. As used herein, the term "or" is generally employed in the sense of "and/or", "several" of "at least one" and "at least two" of "two or more", unless the context clearly dictates otherwise. Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal" generally refers to an end closer to an operator, and the term "distal" generally refers to an end closer to a lesion in a patient. The terms "one end" and "the other end", as well as "proximal end" and "distal end", are generally used to refer to opposing end portions including the opposing endpoints, rather than only to the endpoints. The terms "mounting", "coupling" and "connection" should be interpreted in a broad sense. For example, a connection may be a permanent, detachable or integral connection, or a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location relative to the other element, unless the context clearly dictates otherwise. Those of ordinary skill in the art can understand the specific meanings of the above-mentioned terms herein, depending on their context.

It is an objective of the present invention to overcome the problem of inconvenient replacement of existing leadless pacemakers after the expiry of their service life, by presenting a novel leadless pacemaker, leading component and trailing component.

This invention will be described in greater detail below with reference to the accompanying drawings.

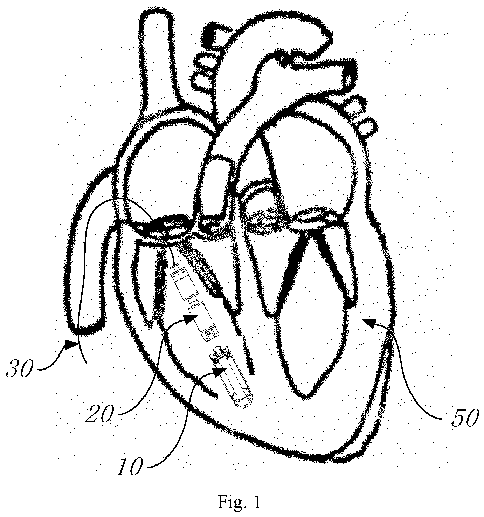

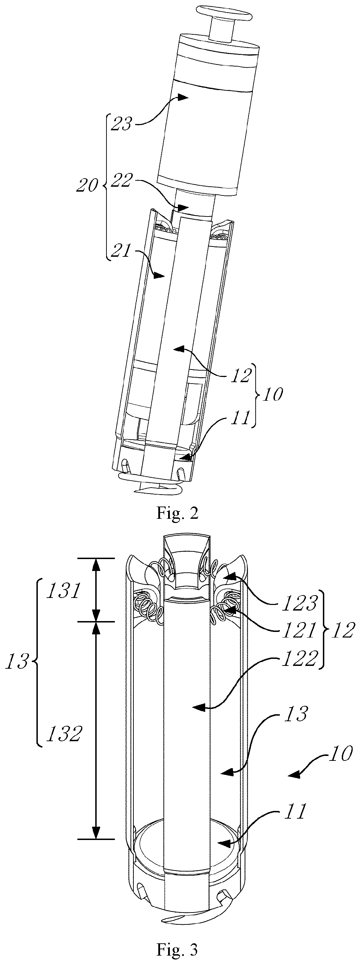

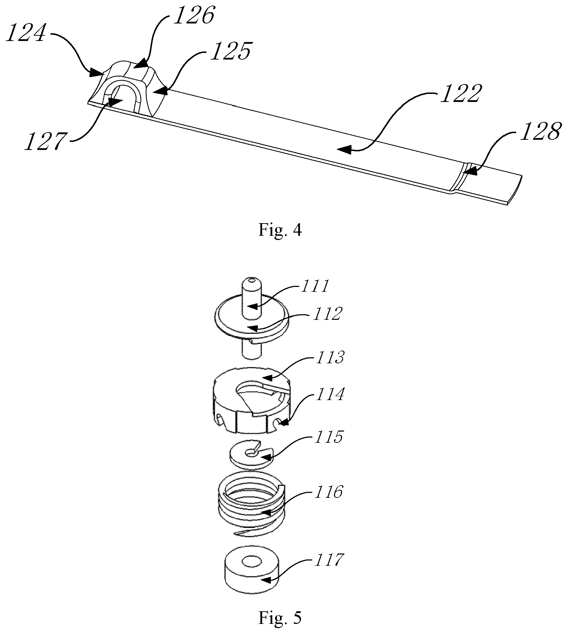

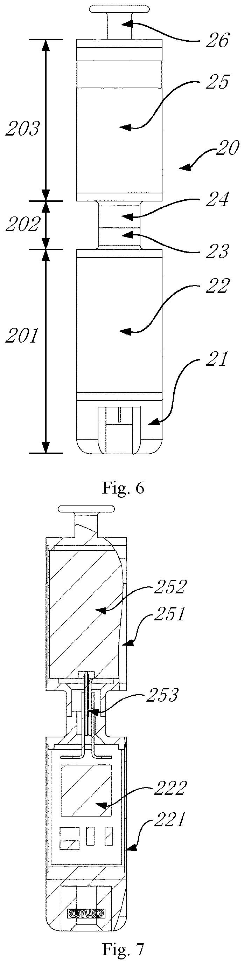

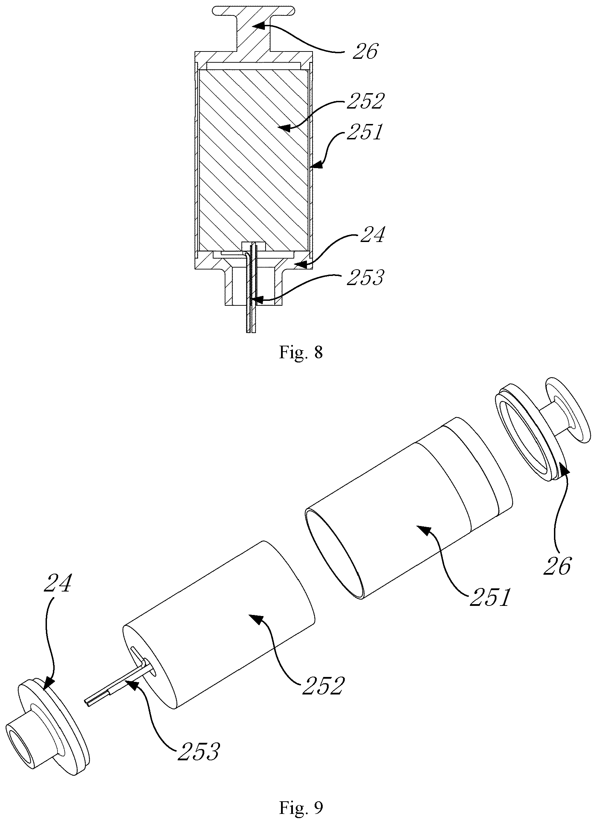

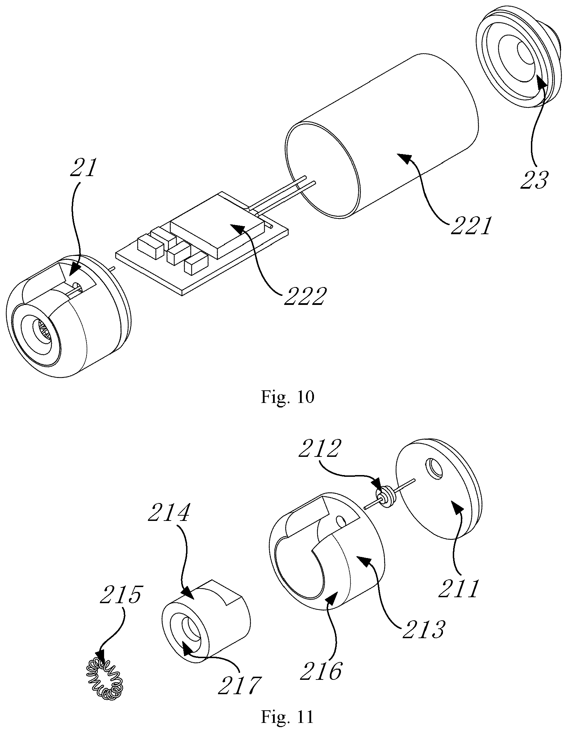

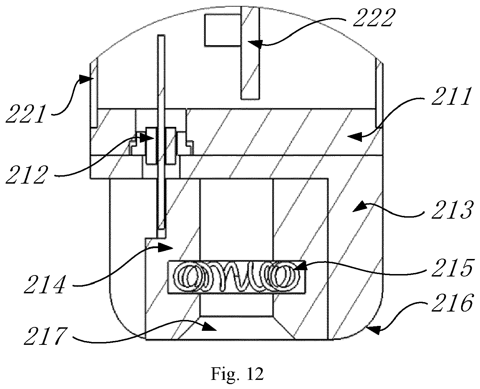

Reference is now made to Figs. 1 to 12. Fig. 1 is a schematic diagram showing an implantation application of a leadless pacemaker according to an embodiment of the present invention. Fig. 2 is a schematic assembly view of a leadless pacemaker according to an embodiment of the present invention. Fig. 3 is a schematic illustration of a leading component according to an embodiment of the present invention. Fig. 4 schematically illustrates resilient strips and resilient element connectors according to an embodiment of the present invention. Fig. 5 is a schematic exploded view of a leading body according to an embodiment of the present invention. Fig. 6 is a schematic illustration of a trailing component according to an embodiment of the present invention. Fig. 7 is a schematic partial cross-sectional view of a trailing component according to an embodiment of the present invention. Fig. 8 is a schematic axial cross-sectional view of a battery assembly according to an embodiment of the present invention. Fig. 9 is a schematic exploded view of a battery assembly according to an embodiment of the present invention. Fig. 10 is a schematic exploded view of an electronic assembly according to an embodiment of the present invention. Fig. 11 is a schematic exploded view of an electrode connector according to an embodiment of the present invention. Fig. 12 is a schematic axial cross-sectional view of an electrode connector according to an embodiment of the present invention.

As shown in Figs. 1 and 2, in one embodiment of the present invention, there is provided a leadless pacemaker including a leading component 10 and a trailing component 20. The leading component 10 is detachably coupled to the trailing component 20. Fig. 1 shows an exemplary embodiment, in which a surgical procedure is carried out using the leadless pacemaker. In this procedure, the leading component 10 may be first implanted in the left ventricular apex of the heart 50 at a pacing site. In this step, an effective pacing site may be identified according to a conventional method for implanting an electrode lead of a pacemaker. After that, the trailing component 20 may be delivered into the left ventricle by a delivery device 30, and then the trailing component 20 is coupled to and assembled with the leading component 10 to form the complete leadless pacemaker, thus achieving the implantation of the leadless pacemaker. When the service life of the leadless pacemaker expires, the leading component 10 may be kept anchored to the heart 50, and the delivery device 30 may be utilized to separate the trailing component 20 from the leading component 10 and withdraw the trailing component 20 from the patient's body. The trailing component 20 may be then replaced with a new one, and the new trailing component 20 may be delivered into the left ventricle and assembled with the leading component 10 remaining therein to again form the complete leadless pacemaker. With this arrangement, the leading component 10 that may have been wrapped by proliferating tissue can be reused and avoided from being disturbed, and it is made unnecessary to create a new incision in the heart. Those skilled in the art can choose a suitable delivery device 30 according to common general knowledge in the art for delivering and implanting the trailing component 20 and the leading component 10, and the present invention is not limited to any particular structure or mechanism of the delivery device 30.

Referring to Figs. 3 and 6, in conjunction with Fig. 2, the leading component 10 includes a leading body 11 and a locking member 12, which are coupled to each other. The leading body 11 is adapted to be secured at a predetermined site (e.g., the left ventricular apex of the heart 50). The locking member 12 assumes an initial configuration when there is no external force acting thereon. Moreover, at least part of the locking member 12 is expandable or contractible radially with respect to the leading body 11 under the action of an external force. The locking member 12 defines an accommodating chamber 13 extending in an axial direction of the leading body 11. The accommodating chamber 13 includes a main portion 132 and a diametrically-reduced portion 131 located proximally with respect to the main portion 132 in the axial direction of the leading body 11. In the initial configuration of the locking member 12, an inner radial dimension of the diametrically-reduced portion 131 is smaller than an inner radial dimension of the main portion 132. Moreover, the diametrically-reduced portion 131 is adapted to accommodate a waist section 202 of the trailing component 20, and the main portion 132 is adapted to accommodate a first shoulder section 201 of the trailing component 20.

The trailing component 20 includes a waist section 202 and a first shoulder section 201, which are joined to each other, the first shoulder section 201 is located at the distal end of the waist section 202. An outer radial dimension of the waist section 202 is smaller than an outer radial dimension of the first shoulder section 201. The first shoulder section 201 is adapted to pass through the diametrically-reduced portion 131 and be inserted and accommodated in the main portion 132 of the leading component 11. It is also adapted to be removed from the main portion 132 through the diametrically-reduced portion 131. The waist section 202 is adapted to be inserted and accommodated in the diametrically-reduced portion 131 of the leading component 11 and removed from the diametrically-reduced portion 131.

In order to make full use of delivery capacity of a delivery channel, the leading component 10 and the trailing component 20 are preferably cross-sectionally circular or polygonal (more preferably regular polygonal). Accordingly, the main portion 132 and the diametrically-reduced portion 131 are also preferably cross-sectionally circular or polygonal. It is to be noted that, here, the inner radial dimensions of the diametrically-reduced portion 131 and the locking member 12 refer to their minimum radial widths measured along a line passing through the axis of the leading body 11. In case of the diametrically-reduced portion 131 and the locking member 12 being cross-sectionally circular, they are diameters of the circular cross-sections, i.e., inner diameters of the diametrically-reduced portion 131 and the locking member 12. In case of the diametrically-reduced portion 131 and the locking member 12 being cross-sectionally polygonal, they are diameters of inscribed circles of the polygonal cross-sections. Likewise, the outer radial dimensions are outer diameters of the circular cross-sections or diameters of circumscribed circles of the polygonal cross-sections. The inner and outer radial dimensions described below are defined in a similar manner.

Due to the locking member 12 in the leading component 10, the trailing component 20 can be conveniently inserted into or removed from the accommodating chamber 13. In this way, the trailing component 20 can be replaced almost in a non-invasive fashion, solving the problem of inconvenient replacement of existing leadless pacemakers after the expiry of their service life. Moreover, since the locking member 12 is radially contractible, during delivery of the leading component 10, the locking member 12 can be crimped by a sheath tube of the delivery device 30, allowing the leading component 10 to have a reduced maximum outer diameter and hence increased passage ability. Additionally, the diametrically-reduced portion 131, the main portion 132, the waist section 202 and the first shoulder section 201 can be assembled together in such a manner that there are no protrusions on an exterior circumferential surface of the trailing component 20, which may cause damage to a blood vessel. As a result, the trailing component 20 can be delivered without use of a protective sleeve, resulting in an additional increase in the passage ability of the trailing component 20.

With continued reference to Fig. 3, in conjunction with Fig. 4, optionally, the locking member 12 may include a first resilient element 121, a plurality of resilient strips 122 and a plurality of resilient element connector 123. The resilient strips 122 may be arranged circumferentially around the leading body 11. The distal ends of the resilient strips 122 are fixedly connected to the leading body 11. The resilient element connectors 123 may be arranged internally with respect to the resilient strips 122, and adjacent resilient element connectors 123 may be connected by the first resilient element 121. The resilient element connectors 123 may together define the diametrically-reduced portion 131, and portions of the resilient strips 122 located distally with respect to the resilient element connectors 123 may define the main portion 132. In a specific example of the locking member 12 shown in Fig. 3, the locking member 12 includes four resilient strips 122 and four resilient element connectors 123. The distal ends of the resilient strips 122 are fixed to a circumferential side wall of the leading body 11 and the proximal ends of the resilient strips 122 are free. When driven by an external force, the proximal ends of the resilient strip 122 may be deflected radially with respect to the leading body 11. Preferably, the four resilient strips 122 are evenly arranged circumferentially around the leading body 11 and together define the accommodating chamber 13. The inner side of each resilient strip 122 (which is a side of the resilient strips 122 facing toward the axis of the leading body 11) is fixedly with a respective one of the resilient element connectors 123. It would be appreciated that a radial extent of the portion of the accommodating chamber 13 in which the resilient element connectors 123 are arranged is smaller than that of the portion of the accommodating chamber 13 defined by the other portions of the resilient strips 122 than those provided thereon with the resilient element connectors 123, due to occupation by the resilient element connectors 123. This is the reason why the portion of the accommodating chamber 13 in which the resilient element connectors 123 are arranged is designated as the diametrically-reduced portion 131 and the portion defined by the resilient strip portions 122 located distally with respect to the resilient element connectors 123 as the main portion 132. The present invention is not limited to any particular axial positions of the resilient element connectors 123 on the resilient strips 122. For example, the resilient element connectors 123 may be provided at proximal ends of the resilient strips 122 (see Fig. 3), or the resilient element connectors 123 may be provided at certain distances away from the proximal ends of the resilient strips 122. Additionally, the axial positions of the resilient element connectors 123 corresponding to different resilient strips 122 may be the same or different. Preferably, referring to Fig. 3, in a preferred example, the four resilient element connectors 123 may be provided at the proximal ends of the respective four resilient strips 122, and the four resilient element connectors 123 may be sized, structured and axially positioned in the same way. This arrangement can facilitate both maximized utilization of the accommodating chamber 13 defined by the resilient strips 122 and uniform stressing of the locking member 12.

In an alternative embodiment, the first resilient element 121 may be a spring or the like, for example. Moreover, either a single first resilient element 121, or a plurality of first resilient elements 121 may be provided. For example, a single first resilient element 121 may be provided and successively connected to all the resilient element connectors 123. In case of a plurality of first resilient elements 121, each adjacent two of the resilient element connectors 123 may be connected by one of the first resilient elements 121 disposed therebetween. In these cases, similar effects can be achieved. In a preferred example, one first resilient element 121 is provided, and each of the resilient element connectors 123 defines a through bore 127 extending therethrough in the circumferential direction of the leading body 11, the through bore 127 is configured for passage of the first resilient element 121 therethrough. The first resilient element 121 is shaped like a ring and successively passed through the through bores 127 of the resilient element connectors 123. In this way, adjacent resilient element connectors 123 are connected by the first resilient element 121, and when external forces are exerted on the resilient element connectors 123 to bias them toward a radially expanded configuration (e.g., as a result of passage of the first shoulder section 201 through the diametrically-reduced portion 131), portions of the resilient strips 122 provided thereon with the resilient element connectors 123 are outwardly deflected evenly while being subjected to resilient forces of the first resilient element 121. After the first shoulder section 201 passes through the diametrically-reduced portion 131 and enters the main portion 132, the portions of the resilient strips 122 provided thereon with the resilient element connectors 123 inwardly return evenly under the resilient forces of the first resilient element 121. Preferably, in the initial configuration of the locking member 13, the inner radial dimension of the diametrically-reduced portion 131 is smaller than the outer radial dimension of the waist section 202. In this way, once the first shoulder section 201 enters the main portion 132, the inner side surfaces of the resilient element connectors 123 can fit on the waist section 202, preventing the radial displacement of the trailing component 20 relative to the leading component 10. Moreover, the resilient element connectors 123 can restrict a proximal end of the first shoulder section 201, locking the first shoulder section 201 within the main portion 132. In order to remove the trailing component 20, apply a force towards the proximal end to the trailing component 20 through the delivery device 30, causing the first shoulder section 201 to overcome the resilient forces of the first resilient element 121 to move proximally from the main portion 132 into the diametrically-reduced portion 131 and then out of the leading component 10.

Additionally, the first resilient element 121 disposed between the resilient element connectors 123 can provide a ring-shaped physical barrier, the first resilient element 121 can block the proximal end of the first shoulder section 201 after the first shoulder section 201 is received in the main portion 132. That is, the first resilient element 121 can abut against the proximal end of the first shoulder section 201, enabling the locking member 12 to more reliably lock the first shoulder section 201.

Preferably, with continued reference to Fig. 4, in conjunction with Fig. 3, each resilient element connector 123 includes a first bevel 124 and a second bevel 125, the first bevel 124 faces toward the proximal end, and the second bevel 125 faces toward the distal end, the first bevel 124 and the second bevel 125 are both inclined inwardly toward the axis of the leading body 11. Here, by "inclined inwardly toward the axis of the leading body 11", it is intended to mean that, when the resilient strips 122 are assembled with the leading body 11, the normal of the first bevel 124 and the normal of the second bevel 125 all obliquely point toward the axis of the leading body 11 (i.e., toward the interior of the leading component 10). It is to be noted that an angle of inclination of the first bevel 124 relative to the axis of the leading body 11 may be equal to or different from an angle of inclination of the second bevel 125 relative to the axis of the leading body 11. The first bevel 124 and the second bevel 125 enable the first shoulder section 201 to pass through the diametrically-reduced portion 131 at a smaller angle of interaction between the first shoulder section 201 and the resilient element connectors 123, thereby reducing the frictional resistance of the first shoulder section 201 passing through the diametrically-reduced portion 131. Each resilient element connector 123 further includes a first transition surface 126, the first transition surface 126 is joined to both the first bevel 124 and the second bevel 125. For example, these joints may be rounded and smoothed, or chamfered. In one embodiment, the first transition surface 126 may include a straight section arranged radially with respect to the leading body 11 and two rounded sections respectively located proximally and distally with respect to the straight sections. The two rounded sections may be smoothly joined to the first bevel 124 and the second bevel 125 in order to additionally reduce frictional resistance that the first shoulder section 201 encounters during its passage through the diametrically-reduced portion 131. Optionally, each resilient strip 122 may include a folding section 128, the folding section 128 is preferably located proximally with respect to the joint of the specific resilient strip 122 with the leading body 11. These folding sections can improve the radial strength of the resilient strips 122 and the fatigue resistance of the resilient strips 122, thereby extending the service life of the leading component 10 by enabling it to resist more mating/unmating cycles with trailing components 20.

In practical use, forces required to insert and remove the first shoulder section 201 into and from the diametrically-reduced portion 131 may be measured. These insertion and removal force may be modified by adjusting the size of the first resilient element 121 and the angles of the first bevel 124 and the second bevel 125. Considering the weight of the trailing component and tensile resistance of the myocardium (about 2 N), the insertion force may be determined as 0.3 N ± 0.2 N, and the removal force as 0.5 N ± 0.2 N (as detailed below, the removal force may further depend on a second resilient element 215 and an electrode head 111).

Optionally, referring to Fig. 5, the distal end of the leading body 11 may be provided with a pointed helix 116 and the proximal end of the leading body 11 may be provided with the electrode head 111, the electrode head 111 is electrically connected to the pointed helix 116. The electrode head 111 is configured for electrical connection with an electrode connector 21 in the trailing component 20. The pointed helix 116 may be rotated and thereby screwed into tissue of the heart 50 at a predetermined site, thus anchoring the leading body 11 within the heart 50. In an alternative embodiment, the leading body 11 may further include an electrode 112, a stop ring 113, a snap ring 115 and a drug plunger 117. The electrode head 111 is disposed at a proximal end of the electrode 112 and is configured for electrical connection with the trailing component 20. The stop ring 113 defines several stop notches 114, the pointed helix 116 can snap in the stop notches 114, and the snap ring 115 snaps at a distal end of the electrode 112. In this way, the electrode 112 can be fixed to the stop ring 113 and prevented from dislodgement of the electrode 112 as a result of loosening of the pointed helix 116. Preferably, the electrode 112 and the electrode head 111 are made of stainless steel or platinum-iridium alloy, and the stop ring 113 is made of stainless steel or pure titanium. Moreover, the snap ring 115 is made of an insulating material, which enables the snap ring 115 to insulate the electrode 112 from the stop ring 113 in addition to providing the snap-fastening function. The pointed helix 116 is made of stainless steel 316L or MP35N alloy and adapted to be screwed into the myocardium and cooperate with the stop ring 113 to anchor the leading body 11 to endocardium of the heart 50. Further, the drug plunger 117 contains silica gel and dexamethasone acetate as main ingredients and is used for hemostasis and inflammation suppression of the screwed portion of the endocardium. Those skilled in the art would recognize how the leading body 11 is structured based on common general knowledge in the art. It is to be noted that the leading body 11 shown in Fig. 5 is merely an example, and its structure is not limited as shown in the figure. Those skilled in the art can choose any other suitable conventional structure for the leading body 11, as long as it can be anchored in the heart 50 at the predetermined site. For example, the pointed helix may be omitted, and the leading body 11 may instead include self-releasable tines or the like while still achieving similar effects. The present invention is not limited in this regard.

Preferably, the electrode head 111 is columnar and extends in the axial direction of the leading body 11. In one example, the electrode head 11 is substantially cylindrical and disposed on the axis of the leading body 11. The proximal end of the electrode head 111 may define a hemispherical guide structure for facilitating it connection with the electrode connector 21 in the trailing component 20. The pointed helix 116 (or self-releasable tines, etc.) is generally a metal element, which, when screwed into the heart 50 at the predetermined site, can establish an electrical connection with the heart 50, thereby allowing sensing of heartbeats (capture of sense signals) and delivery of impulses (pacing signals) to the heart. The electrode head 111 is electrically connected to the pointed helix 116 and functions as a conductor for transmitting the sense signals to the trailing component 20 and the pacing signals to the pointed helix 116.

According to the leading component 10 of this embodiment, since the proximal ends of the resilient strips 122 in the locking member 12 can be radially deflected inwardly, during delivery of the leading component 10, the locking member 12 can be crimped by a sheath tube so that an outer diameter of the leading body 11 defines a maximum outer diameter of the leading component 10 during passage. On the other hand, the structure of the locking member 12 has no impact on the maximum outer diameter of the leading component 10 during passage. This allows convenient assembly and disassembly of the locking member 12, while ensuring passage ability of the leading component 10. Further, it is ensured that the forces for inserting and removing the first shoulder section 201 into and from the locking member 12 are measureable and consistent, enabling implantation and retrieval of the trailing component 20 to be accomplished almost in a non-invasive fashion.

The trailing component 20 in this embodiment will be described in greater detail below by way of an example with reference to Figs. 6 to 12. Referring to Figs. 6 and 7, optionally, the trailing component 20 may include a battery assembly 25, an electronic assembly 22 and the electrode connector 21. The electrode connector 21 is configured for electrical connection with the electrode head 111 in the leading component 10, and the electrode connector 21 is electrically connected to the electronic assembly 22. The electronic assembly 22 essentially includes a circuit module 222 for the leadless pacemaker, which is capable of acquiring the sense signals from the leading component 10 and sending the pacing signals to the leading component 10. The battery assembly 25 is adapted to provide a power supply for the electronic assembly 22. Expiry of the service life of a battery in the battery assembly 25, or that of the circuit module 222 in the electronic assembly 22, may be considered as expiry of the service life of the entire trailing component 20. When this happens, the trailing component 20 may be replaced as a whole.

In an alternative embodiment, the battery assembly 25, the electronic assembly 22 and the electrode connector 21 are sequentially joined end-to-end from a proximal end to a distal end of the trailing component 20. Referring to Fig. 10, the electronic assembly 22 includes a first casing 221, the circuit module 222 is accommodated in the first casing 221. The circuit module 222 is connected by a power cable 253 to the battery in the battery assembly 25 in order to be powered thereby. Moreover, the circuit module 222 is also electrically connected to the electrode connector 21 in order to exchange the sense and pacing signals with the leading component 10. The proximal end of the first casing 221 is sealed by a first cover 23 and the distal end of the first casing 221 is connected sealingly to the electrode connector 21. Preferably, the first casing 221 and the first cover 23 are each made of a metal material such as pure titanium or stainless steel.

Referring to Figs. 8 and 9, the battery assembly 25 includes a second casing 251, the battery 252 is accommodated in the second casing 251. The distal end of the second casing 251 is sealed by a second cover 24. One end of the power cable 253 is connected to the battery 252 and passed successively through the second cover 24 and the first cover 23 and connected to the circuit module 222. Preferably, the second casing 251 and the second cover 24 are each made of a metal material such as pure titanium or stainless steel. The battery 252 is preferably a primary lithium battery sprayed on its outer surface with parylene for insulation, and the power cable 253 is preferably a flexible metal wire covered with an insulating jacket.

Referring to Figs. 11 and 12, the electrode connector 21 is adapted to be electrically connected to the electrode head 111 in the leading body 11. The electrode connector 21 includes a third cover 211, a feedthrough 212, an intermediate element 213, a square body 214 and the second resilient element 215. The proximal end of the third cover 211 is adapted to be connected sealingly to (e.g., by welding) the first casing 221. The distal end of the third cover 211 is coupled to the intermediate element 213. The distal end of the intermediate element 213 defines a square body accommodation cavity, in which the square body 214 can be secured.

Preferably, the electrode connector 21 includes an electrode chamber extending along an axial direction of the first shoulder section 201, the electrode chamber is open distally and adapted to accommodate the electrode head 111. In the example shown in Figs. 11 and 12, the electrode chamber is defined at a distal end of the square body 214, and the second resilient element 215 circumferentially surrounds the electrode chamber. Optionally, the second resilient element 215 may be, for example, a spring or another element with resilience. The second resilient element 215 is adapted to abut against and thereby be electrically connected to the electrode head 111. More preferably, the second resilient element 215 is ring-shaped, the second resilient element 215 has an inner radial dimension smaller than an outer radial dimension of the electrode head 111. With this arrangement, when the electrode head 111 is inserted in the second resilient element 215, the second resilient element 215 will be in a radially expanded configuration with good electrical contact. Optionally, the second resilient element 215 may be made of MP35N alloy. The dimensional relationship between the second resilient element 215 and the electrode head 111 has an impact on the aforementioned removal force. Those skilled in the art can properly configure the dimensional relationship between the second resilient element 215 and the electrode head 111 according to the removal force range listed above. Preferably, a third bevel 217 is formed at the distal opening of the electrode chamber in order to guide the insertion of the electrode head 111.

Preferably, the third cover 211 and the square body 214 are each made of a metal material such as pure titanium or stainless steel. Preferably, the intermediate element 213 is made of an insulating material such as a plastic in order to electrically insulate the square body 214 from the third cover 211. Feedthrough bores for assembly of the feedthrough 212 are defined in the third cover 211 and the intermediate element 213 at corresponding locations. The feedthrough 212 is substantially structured as a needle-shaped conductor surrounded by a circular insulating coil. The coil is mounted in the feedthrough bores, without direct contact between the needle-shaped conductor and the feedthrough bores. The distal end of the needle-shaped conductor is electrically connected to the square body 214. For example, the two may be brought into contact with each other to establish an electrical connection between them. The proximal end of the needle-shaped conductor is passed through the third cover 211 into the first casing 221 and electrically connected to the circuit module 222 therein. It would be appreciated that the sense signals propagate successively through the pointed helix 116, the electrode head 111, the second resilient element 215, the feedthrough 212 (more precisely, the needle-shaped conductor therein) and the circuit module 222, and the pacing signals propagate through a reverse path.

The trailing component 20 further includes a delivery connector 26, the delivery connector 26 is disposed at a proximal end of the trailing component 20 and configured for connection of the delivery device 30. With continued reference to Figs. 8 and 9, in one embodiment, the delivery connector 26 is disposed at a proximal end of the battery assembly 25 so as to be sealingly connected to the second casing 251. The delivery connector 26 may have, for example, a T-shaped axial cross-section, which can facilitate connection of the delivery device 30.

Optionally, the first cover 23 may be a diameter-varying structure including a distal sealing section and a proximal coupling section. An outer radial dimension of the sealing section matches an outer radial dimension of the first casing 221 so that the sealing section can be sealingly assembled with the first casing 221. Preferably, after the first cover 23 is assembled with the first casing 221, the first cover 23 is not radially raised over the first casing 221 at all. More preferably, radial contours of the sealing section and the first casing 221 are both circular and have equal outer diameters. An outer radial dimension of the coupling section is smaller than the outer radial dimension of the sealing section. Preferably, the coupling section also has a circular radial contour. The second cover 24 may have a similar structure to that of the first cover 23 but assumes an opposite orientation. That is, the second cover 24 includes a distal coupling section and a proximal sealing section. The sealing section of the second cover 24 is adapted to be sealingly assembled with the second casing 251. Preferably, the coupling section of the second cover 24 has the same radial contour as the coupling section of the first cover 23, and the two are coupled to each other (e.g., by laser welding) in such a manner that the joint is straight without any radially raised portion. Preferably, the second casing 251 also has a circular radial contour and the same outer diameter as that of the first casing 221. More preferably, the first casing 221, the first cover 23, the second cover 24 and the second casing 251 are coaxially assembled together end-to-end. Further, the electrode connector 21 also has a circular radial contour and the same outer diameter as that of the first casing 221.

In this example of the trailing component 20, the coupling sections of the first cover 23 and the second cover 24 make up the waist section 202, and the sealing section of the first cover 23, the first casing 221 and the electrode connector 21 make up the first shoulder section 201. In this way, the trailing component 20 includes at least the first shoulder section 201 and the waist section 202 that are coupled to each other. The first shoulder section 201 is located distally with respect to the waist section 202, and the outer radial dimension of the waist section 202 is smaller than the outer radial dimension of the first shoulder section 201. The first shoulder section 201 is adapted to be inserted through the diametrically-reduced portion 131 into the main portion 132 of the leading component 10 and removed from the main portion 132 through the diametrically-reduced portion 131. The waist section 202 is adapted to be inserted and accommodated in the diametrically-reduced portion 131 of the leading component 10 and removed from the diametrically-reduced portion 131. It would be appreciated that, with reference to the above description in connection with the leading component 10, as a result of the distal first shoulder section 201 of the trailing component 20 being inserted into the diametrically-reduced portion 131 of the leading component 10, the diametrically-reduced portion 131 will be expanded. Moreover, when the first shoulder section 201 completely passes through the diametrically-reduced portion 131 and enters the main portion 132, the diametrically-reduced portion 131 will contract until it abuts against and fastens the waist section 202. In this way, the trailing component 20 can be assembled with the leading component 10. The trailing component 20 can be disassembled from the leading component 10 by a reverse process, further description of which is, however, omitted herein.

Preferably, referring to Fig. 11, the first shoulder section 201 includes a second transition surface 216, which is joined to both a circumferential side wall of the first shoulder section 201 and a distal end face of the first shoulder section 201. In the example shown in Fig. 11, the second transition surface 216 is provided on the intermediate element 213 and preferably has a smooth outer contour shape for reducing resistance that the first shoulder section 201 encounters during its passage through the diametrically-reduced portion 131.

Preferably, the trailing component 20 further includes a second shoulder section 203 coupled to a proximal end of the waist section 202. An outer radial dimension of the second shoulder section 203 is greater than the outer radial dimension of the waist section 202. Referring to Fig. 6, in one example, the second shoulder section 203 is made up of the sealing section of the second cover 24, the second casing 251 and part of the delivery connector 26. Optionally, the outer radial dimension of the second shoulder section 203 may be smaller than the outer radial dimension of the first shoulder section 201. In order to make full use of a radial size of the delivery channel, the outer radial dimension of the second shoulder section 203 is preferably adapted to be equal to the outer radial dimension of the first shoulder section 201. In this way, the second casing 251 can have a larger internal space capable of accommodating a battery with a larger capacity. This embodiment is not limited to any particular outer radial dimension of the second shoulder section 203, and the outer radial dimension of the second shoulder section 203 may even be adapted to equate the outer radial dimension of the waist section 202. In this case, reliable assembly and disassembly can be still attained between the trailing component 20 and the leading component 10.

With the above arrangement, the trailing component 20 assumes a dumbbell-like shape, which is wider at both ends and narrower in the middle, and has no radial protrusions that may cause damage to a blood vessel during delivery therein. Therefore, it is made unnecessary for the trailing component 20 to be protected with a sleeve during its delivery. Accordingly, at a given delivery size, an even greater internal space can be achieved to accommodate a battery with an even larger capacity, which can additionally extend the service life.

It is to be noted that Figs. 6 to 12 illustrate only one example of the trailing component 20, and the trailing component 20 is not limited as shown in the figures. In practice, the electrode connector 21, the electronic assembly 22 and the battery assembly 25 are not limited to being sequentially arranged from the distal end to the proximal end. For example, in some embodiments, the electronic assembly 22 and the battery assembly 25 may be interchanged in position, or the electronic assembly 22 and the battery assembly 25 may be integrated into a single component, or the battery 252 and the circuit module 222 may be both accommodated in the first casing 221 so that the three together make up the first shoulder section 201. Those skilled in the art may make various modifications within the scope of the foregoing teachings, and the present invention is not limited in this regard.

In summary, the present invention provides a leadless pacemaker, a leading component and a trailing component. The leading component includes a leading body and a locking member, which are coupled to each other. The leading body is adapted to be secured to a predetermined object. The locking member assumes an initial configuration when there is no external force acting thereon. At least part of the locking member is expandable or contractible radially with respect to the leading body under the action of an external force. The locking member defines an accommodating chamber extending in an axial direction of the leading body. The accommodating chamber includes a main portion and a diametrically-reduced portion located at a proximal end of the main portion in the axial direction of the leading body. In the initial configuration of the locking member, an inner radial dimension of the diametrically-reduced portion is smaller than an inner radial dimension of the main portion. The diametrically-reduced portion is adapted to accommodate a waist section of the trailing component, and the main portion is adapted to accommodate a first shoulder section of the trailing component.

With this arrangement, the leading component and the trailing component are assembled in a detachable and separable manner. During use, the leading component may be first implanted within the heart at a predetermined site, and the trailing component may be delivered into the heart and coupled to and assembled with the leading component. When the service life of the leadless pacemaker expires, the trailing component may be disassembled from the leading component and retrieved, with the leading component being retained at the predetermined site. After that, the trailing component may be replaced with a new one. The locking member in the leading component enables the trailing component to be conveniently inserted into or removed from the accommodating chamber. In this way, the trailing component can be replaced almost in a non-invasive fashion, solving the problem of inconvenient replacement of existing leadless pacemakers after the expiry of their service life. Additionally, since the locking member is radially contractible, during delivery of the leading component, the locking member can be crimped by a sheath tube, allowing the leading component to have a reduced maximum outer diameter and hence increased passage ability. Additionally, the diametrically-reduced portion, the main portion, the waist section and the first shoulder section can be assembled together in such a manner that there are no protrusions on an exterior circumferential surface of the trailing component, which may cause damage to a blood vessel. As a result, the trailing component can be delivered without use of a protective sleeve, resulting in an additional increase in the passage ability of the trailing component.

It is to be noted that the foregoing embodiments are merely exemplary of the present invention and do not limit the scope thereof in any way. Although various variations and modifications can be made by those of ordinary skill in the art without departing from the concept of the present invention, any and all such variations and modifications are intended to be embraced within the scope of the invention as defined by the appended claims.

## Claims

1. A leading component of a leadless pacemaker, the leading component adapted to be detachably coupled to a trailing component of a leadless pacemaker, the leading component comprising a leading body and a locking member, which are connected to each other,
the leading body configured to be secured at a predetermined site,
the locking member assuming an initial configuration when there is no external force acting thereon, at least part of the locking member expandable or contractible radially with respect to the leading body under the action of an external force,
the locking member defining an accommodating chamber extending in an axial direction of the leading body, the accommodating chamber comprising a main portion and a diametrically-reduced portion located at a proximal end of the main portion in the axial direction of the leading body, the diametrically-reduced portion having an inner radial dimension smaller than an inner radial dimension of the main portion when the locking member is in the initial configuration, the diametrically-reduced portion configured to accommodate a waist section of the trailing component, the main portion configured to accommodate a first shoulder section of the trailing component.

2. The leading component of claim 1, wherein the locking member comprises a first resilient element, a plurality of resilient strips and a plurality of resilient element connectors, the plurality of resilient strips arranged circumferentially around the leading body, distal ends of the resilient strips fixedly connected to the leading body, the resilient element connectors provided on inner sides of the resilient strips, each adjacent two of the resilient element connectors are connected by the first resilient element, the plurality of the resilient element connectors defining the diametrically-reduced portion, portions of the plurality of resilient strips located at distal ends of the resilient element connectors defining the main portion.

3. The leading component of claim 2, wherein each of the resilient element connectors comprises a first bevel and a second bevel, the first bevel facing proximally, the second bevel facing distally, both the first bevel and the second bevel inclined toward an axis of the leading body.

4. The leading component of claim 3, wherein each of the resilient element connectors further comprises a first transition surface, the first transition surface transitionally joined to both the first bevel and the second bevel.

5. The leading component of claim 2, wherein each of the resilient element connectors defines a through bore extending therethrough in the circumferential direction of the leading body, the through bore configured for passage of the first resilient element therethrough, wherein the first resilient element is ring-shaped and passed successively through the through bores of the plurality of the resilient element connectors.

6. The leading component of claim 2, wherein the resilient element connectors are provided on proximal ends of the resilient strips.

7. The leading component of claim 2, wherein three or four resilient strips are provided, the resilient element connectors in one-to-one correspondence with the resilient strips.

8. The leading component of claim 1, wherein a distal end of the leading body defines a pointed helix, wherein a proximal end of the leading body is provided with an electrode head, the electrode head electrically connected to the pointed helix, the electrode head configured for electrical connection with an electrode connector in the trailing component.

9. The leading component of claim 8, wherein the electrode head is columnar and extends in the axial direction of the leading body.

10. The leading component of claim 1, wherein when the locking member is in the initial configuration, the inner radial dimension of the diametrically-reduced portion is smaller than an outer radial dimension of the waist section in the trailing component.

11. A trailing component of a leadless pacemaker, the trailing component adapted to be detachably coupled to a leading component of a leadless pacemaker, the trailing component comprising a waist section and a first shoulder section, which are connected to each other,
the first shoulder section located at a distal end of the waist section, the waist section having an outer radial dimension smaller than an outer radial dimension of the first shoulder section,
the first shoulder section configured to be inserted through a diametrically-reduced portion of the leading component and accommodated in a main portion of the leading component and to be removed from the main portion through the diametrically-reduced portion of the leading component, the waist section configured to be inserted and accommodated in the diametrically-reduced portion of the leading component and to be removed from the diametrically-reduced portion of the leading component.

12. The trailing component of claim 11, wherein the first shoulder section comprises a second transition surface, the second transition surface transitionally joined to both a circumferential side wall of the first shoulder section and a distal end face of the first shoulder section.

13. The trailing component of claim 11, further comprising an electrode connector, the electrode connector configured to be electrically connected to an electrode head of the leading component.

14. The trailing component of claim 13, wherein the electrode connector comprises an electrode chamber extending in an axial direction of the first shoulder section, the electrode chamber open distally and configured to accommodate the electrode head of the leading component.

15. The trailing component of claim 14, wherein the electrode connector further comprises a second resilient element arranged circumferentially around the electrode chamber, the second resilient element configured to abut against and thereby be electrically connected to the electrode head of the leading component.

16. The trailing component of claim 15, wherein the second resilient element is ring-shaped, the second resilient element having an inner radial dimension smaller than an outer radial dimension of the electrode head of the leading component.

17. The trailing component of claim 11, further comprising a second shoulder section, the second shoulder section connected to a proximal end of the waist section, the second shoulder section having an outer radial dimension greater than the outer radial dimension of the waist section.

18. The trailing component of claim 17, wherein the outer radial dimension of the second shoulder section is equal to the outer radial dimension of the first shoulder section.

19. The trailing component of claim 11, further comprising a delivery connector, the delivery connector disposed at a proximal end of the trailing component and configured for connection of a delivery device.

20. A leadless pacemaker, comprising the leading component of any one of claims 1 to 10 and the trailing component of any one of claims 11 to 19, the trailing component configured to be detachably coupled to the leading component,
wherein the outer radial dimension of the waist section is greater than the inner radial dimension of the diametrically-reduced portion when the locking member is in the initial configuration;
when the first shoulder section passes through the diametrically-reduced portion, the first shoulder section pushes and radially expands at least part of the locking member, and after the first shoulder section enters or is proximally removed from the main portion through the diametrically-reduced portion, the at least part of the locking member is radially contractible; and
after the first shoulder section enters the main portion, the waist section is inserted and accommodated in the diametrically-reduced portion.
